# EUROPEAN PATENT SPECIFICATION

(11) **EP 3 040 068 B1**
(45) Date of publication and mention of the grant of the patent: **18.08.2021**
(21) Application number: 14810664.4
(22) Date of filing: 12.06.2014
(51) Int. Cl.: A61K 47/44, A61K 9/70, A61K 31/27

(54) **ADHESIVE SHEET FOR APPLICATION TO THE SKIN, AND PERCUTANEOUS ABSORPTION PREPARATION USING SAME**
KLEBEFOLIE ZUR ANWENDUNG AUF DER HAUT UND PERKUTANES ABSORPTIONSPRÄPARAT DAMIT
FEUILLE ADHÉSIVE À APPLIQUER SUR LA PEAU, ET PRÉPARATION D'ABSORPTION PERCUTANÉE L'UTILISANT

(30) Priority: 12.06.2013 WO PCT/JP2013/066273; 11.12.2013 JP 2013273767
(43) Date of publication of application: 06.07.2016
(73) Proprietor: KM Transderm Ltd., Osaka-shi, Osaka 530-8288 (JP)
(72) Inventor: OGINO, Hiroyuki, Higashikagawa-shi Kagawa 769-2702 (JP); YUKIMOTO, Sadao, Higashikagawa-shi Kagawa 769-2702 (JP); HAMADA, Atsuyo, Higashikagawa-shi Kagawa 769-2702 (JP); AKAZAWA, Mitsuji, Higashikagawa-shi Kagawa 769-2702 (JP); GOTO, Masaoki, Higashikagawa-shi Kagawa 769-2702 (JP)
(74) Representative: dompatent von Kreisler Selting Werner - Partnerschaft von Patent- und Rechtsanwälten mbB
(86) International application number: PCT/JP2014/065637
(87) International publication number: WO 2014/200072

(56) References cited:
- EP-A1- 1 864 655
- WO-A1-2012/029325
- WO-A1-2013/020556
- WO-A1-2013/081014
- JP-A- 2012 149 061
- US-A1- 2006 211 808
- CHENGXIAO WANG ET AL: "Evaluation of Drug Release Profile from Patches Based on Styrene-Isoprene-Styrene Block Copolymer: The Effect of Block Structure and Plasticizer", AAPS PHARMSCITECH, SPRINGER NEW YORK LLC, US, vol. 13, no. 2, 3 April 2012 (2012-04-03), pages 556-567, XP035064305, ISSN: 1530-9932, DOI: 10.1208/S12249-012-9778-3
- CHENGXIAO WANG ET AL: "A Drug-in-Adhesive Matrix Based on Thermoplastic Elastomer: Evaluation of Percutaneous Absorption, Adhesion, and Skin Irritation", AAPS PHARMSCITECH, SPRINGER NEW YORK LLC, US, vol. 13, no. 4, 8 September 2012 (2012-09-08), pages 1179-1189, XP035148009, ISSN: 1530-9932, DOI: 10.1208/S12249-012-9849-5
- None

## Description

### Technical Field

The present invention relates to an adhesive sheet for adhesion to the skin, which has sufficient adhesiveness and causes low skin irritation, and a transdermal absorption preparation showing low skin irritation and improved drug releaseability. In the following passages the "invention" is to be understood as strictly limited by the scope of the appended claims.

### Background Art

When a drug is to be transdermally absorbed, the drug is added to an adhesive base and the like and formed as an adhesive preparation. In recent years, tapes more superior in the adhesiveness are often used than poultices containing a large amount of water as a constituent component in an adhesive preparation. As an adhesive base for such tapes, a lipophilic adhesive base such as of rubber, acrylic or silicone type and the like is used. Of these, a rubber adhesive base is widely used since additives can be easily blended as compared to other adhesive bases. (patent documents 1 - 3)

However, problems have been pointed out even for a transdermal absorption preparation using a rubber adhesive base such as unensurable sufficient releaseability of a drug, development of skin irritation caused by a tackifier generally added to a transdermal absorption preparation and the like. See in this context especially EP1864655 A1.

Under such circumstances, the present inventors have found that an adhesive sheet for adhesion to the skin, which has sufficient adhesiveness and causes low skin irritation, can be obtained even without using a tackifier by using, as an adhesive base, a thermoplastic elastomer and a large amount of liquid paraffin relative to the elastomer, and that a transdermal absorption preparation having sufficient transdermal absorbability can be obtained by adding a drug or a pharmaceutically acceptable salt thereof to the adhesive sheet (patent document 4). However, since thermoplastic elastomers and liquid paraffin to be particularly used are not limited, adhesive property is sometimes insufficient depending on the formulation.

### [Document List]

### Patent Documents

patent document 1: JP-A-2001-302502
patent document 2: JP-A-9-291028
patent document 3: JP-A-10-316559
patent document 4: WO 2012/029325

### SUMMARY OF THE INVENTION

### Problems to be Solved by the Invention

An object of the present invention is to provide an adhesive sheet for adhesion to the skin, which has various sufficient adhesive properties and causes low skin irritation, and a transdermal absorption preparation showing low skin irritation, sufficient adhesive properties and sufficient drug releaseability.

### Means of Solving the Problems

The present inventors have conducted intensive studies in an attempt to solve the aforementioned problems and found that an adhesive sheet for adhesion to the skin, which has various sufficient adhesive properties and causes low skin irritation, can be obtained, even without using a tackifier, by using, as an adhesive base, a thermoplastic elastomer which is a mixture of a triblock copolymer and a diblock copolymer, wherein the content of the diblock copolymer in the mixture is not less than 20 wt%, and a non-volatile hydrocarbon oil at a large amount relative to the elastomer. They have further found that a transdermal absorption preparation having sufficient drug releaseability can be obtained by adding a drug or a pharmaceutically acceptable salt thereof to the adhesive sheet.

That is, the present disclosure relates to the following [1] - [12].
[1] An adhesive sheet for adhesion to the skin, comprising an adhesive layer formed on a support, wherein
   the adhesive layer comprises
   at least a thermoplastic elastomer, and
   a non-volatile hydrocarbon oil in an amount exceeding 50 parts by weight and not more than 800 parts by weight per 100 parts by weight of the elastomer, and
   the thermoplastic elastomer is a mixture of a triblock copolymer and a diblock copolymer, wherein the content of the diblock copolymer in the mixture is not less than 20 wt%.
[2] The adhesive sheet of the above-mentioned [1], wherein a 25 wt% toluene solution of the thermoplastic elastomer has a solution viscosity of not less than 0.5 Pa·s at 25°C.
[3] The adhesive sheet of the above-mentioned [1] or [2], wherein the thermoplastic elastomer is a styrene-based block copolymer.
[4] The adhesive sheet of the above-mentioned [3], wherein the styrene-based block copolymer is a mixture of a styrene-isoprene-styrene block copolymer and a styrene-isoprene block copolymer.
[5] The adhesive sheet of any one of the above-mentioned [1] - [4], wherein the content of the diblock copolymer in the mixture is not less than 30 wt%.
[6] The adhesive sheet of any one of the above-mentioned [2] - [5], wherein a 25 wt% toluene solution of the thermoplastic elastomer has a solution viscosity of not less than 0.7 Pa·s at 25°C.
[7] The adhesive sheet of any one of the above-mentioned [1] - [6], wherein the non-volatile hydrocarbon oil has a kinematic viscosity of not less than 80 mm²/s at 40°C.
[8] The adhesive sheet of any one of the above-mentioned [1] - [7], wherein the content of the non-volatile hydrocarbon oil in the adhesive layer is not less than 23.5 wt% and not more than 88 wt%.
[9] The adhesive sheet of any one of the above-mentioned [1] - [8], wherein the adhesive layer optionally further comprises a tackifier, and the content of the tackifier in the adhesive layer is not more than 20 wt%.
[10] The adhesive sheet of the above-mentioned [9], wherein the content of the tackifier in the adhesive layer is not more than 10 wt%.
[11] The adhesive sheet of any one of the above-mentioned [1] - [8], wherein the adhesive layer is free of a tackifier.
[12] A transdermal absorption preparation comprising the adhesive sheet of any of the above-mentioned [1] - [11], and a drug or a pharmaceutically acceptable salt thereof, which is contained in the adhesive layer of the sheet.

### Effect of the Invention

The adhesive sheet for adhesion to the skin of the present invention has various, sufficient adhesive properties and causes low skin irritation when adhered to the skin. In addition, the transdermal absorption preparation of the present invention also shows good drug releaseability. Description of Embodiments

The adhesive sheet for adhesion to the skin and the transdermal absorption preparation of the present disclosure characteristically have an adhesive layer formed on a support, wherein the adhesive layer contains a thermoplastic elastomer and a non-volatile hydrocarbon oil in an amount exceeding 50 parts by weight and not more than 800 parts by weight per 100 parts by weight of the elastomer, and the thermoplastic elastomer is a mixture of a triblock copolymer and a diblock copolymer, wherein the content of the diblock copolymer in the mixture is not less than 20 wt%.

The "thermoplastic elastomer" used in the adhesive layer of the present invention is an elastomer having thermoplasticity that shows flowability since it softens by adding heat and returns to a rubbery elastic body by cooling, and various thermoplastic elastomers of urethane, acrylic, styrene, olefin series and the like can be mentioned. In the present invention, moreover, a mixture of a triblock copolymer and a diblock copolymer, wherein the content of the diblock copolymer in the mixture is not less than 20 wt%, is used as the thermoplastic elastomer in order to impart sufficient skin adhesiveness to the adhesive sheet and transdermal absorption preparation. When the mixing ratio of the diblock copolymer is too low, skin adhesiveness tends to decrease. When it is too high, shape retention of the adhesive layer tends to be degraded, which in turn may cause inconveniences on adhesion to the skin, such as adhesive residue on the skin after peeling off and the like. Therefore, the mixing ratio of the triblock copolymer and the diblock copolymer in weight ratio is preferably 20/80 - 75/25, more preferably 30/70 - 70/30.

Particularly, a 25 wt% toluene solution of the thermoplastic elastomer preferably has a solution viscosity of not less than 0.5 Pa·s at 25°C, further preferably not less than 0.7 Pa·s, particularly preferable not less than 0.9 Pa·s, to afford a good balance of the adhesive property of the obtained adhesive sheet and transdermal absorption preparation. While the upper limit of the solution viscosity is not particularly limited, it is preferably not more than 2.0 Pa·s, more preferably not more than 1.8 Pa·s.

As used herein, the "solution viscosity of a 25 wt% toluene solution at 25°C" is a value measured based on the viscosity measurement method of a styrene-isoprene-styrene block copolymer described on page 375 of "Japanese Pharmaceutical Excipients 2003" (published by YAKUJI NIPPO LIMITED.).

As the thermoplastic elastomer, a styrene-based thermoplastic elastomer, particularly, a styrene-based block copolymer is preferably used for simultaneous achievement of sufficient skin adhesiveness and low skin irritation, which is the object of the present invention. Specific examples of the styrene-based block copolymer include styrene-butadiene block copolymer, styrene-butadiene-styrene block copolymer, styrene-isoprene block copolymer, styrene-isoprene-styrene block copolymer, styrene-ethylene/butylene block copolymer, styrene-ethylene/butylene-styrene block copolymer, styrene-ethylene/propylene block copolymer, styrene-ethylene/propylene-styrene block copolymer, styrene-isobutylene block copolymer, styrene-isobutylene-styrene block copolymer and the like. In the above, the "ethylene/butylene" shows a copolymer block of ethylene and butylene, and the "ethylene/propylene" shows a copolymer block of ethylene and propylene. In these styrene-based block copolymers, three or more kinds may be used in combination as long as the mixing ratio of the above-mentioned triblock copolymer and the diblock copolymer is met. That is, one or more kinds each of the triblock copolymer and diblock copolymer can be used.

Among the above-mentioned styrene-based block copolymers, a styrene-isoprene-styrene block copolymer, and a mixture containing a styrene-isoprene block copolymer are particularly preferably used to simultaneously achieve sufficient skin adhesiveness and low skin irritation, and in view of the availability and handling property of products for adhesion. Particularly, a mixture of the styrene-isoprene-styrene block copolymer and the styrene-isoprene block copolymer is preferably used from the aspect of adhesiveness.

For the object of the present invention, a styrene-isoprene-styrene block copolymer preferably has a styrene content of the copolymer of 5 wt% - 60 wt%, more preferably 10 wt% - 50 wt%. In addition, it preferably has a weight average molecular weight as measured by gel filtration chromatography of 20,000 - 500,000, more preferably 30,000 - 300,000. As the styrene-isoprene block copolymer, one having a styrene content of the copolymer of 5 wt% - 50 wt%, more preferably 10 wt% - 40 wt%. In addition, it preferably has a weight average molecular weight as measured by gel filtration chromatography of 10,000 - 500,000, more preferably 20,000 - 300,000. A mixture of the styrene-isoprene-styrene block copolymer and the styrene-isoprene block copolymer preferably has a weight average molecular weight as measured by gel filtration chromatography of 20,000 - 500,000, more preferably 30,000 - 300,000.

As the styrene-isoprene-styrene block copolymer or styrene-isoprene block copolymer, a copolymer produced by a method known per se can be used. Alternatively, as the styrene-isoprene-styrene block copolymer or styrene-isoprene block copolymer, a commercially available product satisfying the above-mentioned property can be used. In addition, a mixture of the styrene-isoprene-styrene block copolymer and the styrene-isoprene block copolymer is also commercially available, and a commercially available product of a mixture of the styrene-isoprene-styrene block copolymer and the styrene-isoprene block copolymer at the above-mentioned mixing ratio, which satisfies the above-mentioned properties, can be preferably used.

Examples of the commercially available product include "KRATON D1161", "KRATON D1163", "KRATON D1113" and "KRATON D1119" manufactured by KRATON POLYMERS, "JSR SIS5229", "JSR SIS5403" and "JSR SIS5505" manufactured by JSR, "Quintac 3421", "Quintac 3433N", "Quintac 3520", "Quintac 3450", "Quintac 3270" manufactured by Nippon Zeon Co., Ltd. and the like. Of these, "KRATON D1163", "KRATON D1113", "JSR SIS5403", "JSR SIS5505", "Quintac 3433N", "Quintac 3520" are preferably used, and "JSR SIS5505", "Quintac 3520" are particularly preferably used from the aspects of the mixing ratio of the above-mentioned triblock copolymer and diblock copolymer, and solution viscosity.

When the content of the thermoplastic elastomer in the adhesive layer is too small, the shape of the adhesive layer is generally difficult to maintain, and when it is too much, the skin adhesiveness becomes insufficient. Therefore, the thermoplastic elastomer content of the adhesive layer in the patch of the present disclosure is preferably not less than 8 wt%, more preferably not less than 10 wt%, still more preferably not less than 12 wt%, further preferably not less than 15 wt%, further more preferably not less than 18 wt%, particularly preferably not less than 20 wt%, particularly still more preferably not less than 24 wt%, most preferably not less than 28 wt%. It is preferably not more than 66 wt%, more preferably not more than 65 wt%, still more preferably not more than 64 wt%, further preferably not more than 49 wt%, further more preferably not more than 39 wt%.

In a more specifically preferable embodiment, the content of the thermoplastic elastomer in the adhesive layer is, for example, 8 wt% - 66 wt%, more preferably 10 wt% - 65 wt%, particularly preferably 12 wt% - 64 wt%.

In the adhesive sheet for adhesion to the skin and transdermal absorption preparation of the present disclosure, the adhesive layer contains a non-volatile hydrocarbon oil.

As the non-volatile hydrocarbon oil, a chain saturated hydrocarbon having a carbon number of about 20 - 40 or a chain unsaturated hydrocarbon having a carbon number of about 20 - 40 is preferable and, for example, liquid paraffin, squalene, squalane, pristane and the like can be mentioned. Particularly, liquid paraffin is more preferable since it is easily available. Liquid paraffin is a mixture of alkanes which are colorless odorless and liquid, and have a carbon number of not less than 20. In the present invention, one that satisfies the standards of the Japanese Pharmacopoeia, US Pharmacopeia and the like, and the like can be preferably used. The non-volatile hydrocarbon oil having high viscosity is preferable, and liquid paraffin having high viscosity is particularly preferably used from the aspect of adhesiveness.

To be specific, the non-volatile hydrocarbon oil preferably shows kinematic viscosity at 40°C of not less than 60 mm²/s, more preferably not less than 70 mm²/s, further preferably not less than 80 mm²/s, particularly preferably not less than 100 mm²/s. While the upper limit of the kinematic viscosity is not particularly limited, it is, for example, preferably not more than 500 mm²/s, more preferably not more than 250 mm²/s, from the aspects of easy handling, easy availability and the like.

As used herein, the "kinematic viscosity" is a value obtained by converting the viscosity (mPa·s), measured according to "the Japanese Pharmacopoeia 16th Edition", General Test Method, "2.53 viscosity measurement method", "Second method rotary viscosimeter method (2.12 single cylinder type rotary viscosimeter (Brookfield viscosimeter)" (page 59), to kinematic viscosity.

The adhesive sheet for adhesion to the skin and transdermal absorption preparation of the present disclosure contains the above-mentioned non-volatile hydrocarbon oil at a weight ratio of more than 50 parts by weight and not more than 800 parts by weight, relative to 100 parts by weight of the thermoplastic elastomer. When the content of the non-volatile hydrocarbon oil relative to 100 parts by weight of the thermoplastic elastomer is more than 800 parts by weight, shape retention of the adhesive layer becomes difficult. On the other hand, when the content of the non-volatile hydrocarbon oil is not more than 50 parts by weight, the adhesive becomes too hard and sufficient skin adhesiveness tends to be unachieved. Particularly, the followability to the moving skin during adhesion becomes poor, sometimes resulting in falling off during application. From such aspect, the content of the non-volatile hydrocarbon oil in the adhesive layer is preferably 51 part by weight - 800 parts by weight, more preferably 60 parts by weight - 600 parts by weight, particularly preferably 70 parts by weight - 500 parts by weight, relative to 100 parts by weight of the thermoplastic elastomer.

Even in this range, when a non-volatile hydrocarbon oil having a low kinematic viscosity, for example, kinematic viscosity of less than 80 mm²/s at 40°C, is used for a thermoplastic elastomer showing a low (particularly less than 0.5 Pa·s) solution viscosity of a 25 wt% toluene solution of the thermoplastic elastomer, and the content of the non-volatile hydrocarbon oil is high, protrusion of the adhesive is observed during preservation and adhesion, and inconveniences such as attachment to packing materials and clothes tend to occur. In such cases, the content of the non-volatile hydrocarbon oil in the adhesive layer is preferably 51 parts by weight - 300 parts by weight, further preferably 60 parts by weight - 250 parts by weight, particularly preferably 70 parts by weight - 200 parts by weight, relative to 100 parts by weight of the thermoplastic elastomer.

The content of the non-volatile hydrocarbon oil in the adhesive layer is preferably not less than 23.5 wt%, more preferably not less than 25 wt%, still more preferably not less than 26.5 wt%, further preferably not less than 35 wt%, further more preferably not less than 45 wt%, and particularly preferably not less than 50 wt%. In addition, it is preferably not more than 88 wt%, more preferably not more than 85 wt%, still more preferably not more than 83 wt%, more preferably not more than 70 wt%, further more preferably not more than 68 wt%.

In the adhesive sheet for adhesion to the skin and transdermal absorption preparation of the present invention, good adhesiveness to the skin can be exhibited when a thermoplastic elastomer and a non-volatile hydrocarbon oil are contained at the above-mentioned contents and content ratio in the adhesive layer, and the adhesive layer may also contain a tackifier as necessary.

Here, the tackifier is a resin generally used widely for conferring skin adhesiveness in the field of patch and examples thereof include rosin-based resin, polyterpene resin, cumarone-indene resin, petroleum-based resin, terpene-phenol resin, alicyclic saturated hydrocarbon resin and the like. One or more kinds can be selected therefrom and used.

However, when a tackifier is contained in the adhesive layer, to reduce skin irritation and the like, the content of the tackifier in the adhesive layer is not more than 2 wt%, and most preferably free of a tackifier. That is, in relation to the skin adhesiveness of the adhesive sheet for adhesion to the skin and transdermal absorption preparation, the content of the tackifier is adjusted according to the kind, content, and content ratio of the thermoplastic elastomer and non-volatile hydrocarbon oil. When sufficient skin adhesiveness is obtained without containing a tackifier, a tackifier is not necessary.

In the present invention, a drug or a pharmaceutically acceptable salt thereof is further added to the adhesive layer of an adhesive sheet for adhesion to the skin to give a transdermal absorption preparation.

The "drug or a pharmaceutically acceptable salt thereof" in the present invention refers to a drug or a salt thereof to be used for transdermal absorption, and is not particularly limited. Examples of the drug include anti-inflammatory agents such as acetaminophen, phenacetin, mefenamic acid, diclofenac sodium, flufenamic acid, aspirin, sodium salicylate, methyl salicylate, glycol salicylate, aminopyrine, alclofenac, ibuprofen, naproxen, flurbiprofen, ketoprofen, amfenac sodium, mepirizole, indomethacin, piroxicam, felbinac and the like; steroidal anti-inflammatory drugs such as hydrocortisone, triamcinolone, dexamethasone, prednisolone and the like; vasodilators such as diltiazem hydrochloride, pentaerythritol tetranitrate, isosorbide nitrate, tradipil, nicorandil, nitroglycerol, prenylamine lactate, molsidomine, amyl nitrite, tolazoline hydrochloride, nifedipine and the like; antiarrhythmic agents such as procaineamide hydrochloride, lidocaine hydrochloride, propranolol hydrochloride, alprenolol hydrochloride, atenolol, nadolol, metoprolol tartrate, ajmaline, disopyramide, mexiletine hydrochloride and the like; antihypertensive agents such as ecarazine hydrochloride, indapamide, clonidine hydrochloride, bunitrolol hydrochloride, labetalol hydrochloride, captopril, guanabenz acetate, mebutamate, bethanidine sulfate and the like; antitussive expectorants such as carbetapentane citrate, cloperastine, oxeladin tannate, cloputinol hydrochloride, clofedanol hydrochloride, noscapine hydrochloride, ephedrine hydrochloride, isoproterenol hydrochloride, cloriprenaline hydrochloride, methoxyphenamine hydrochloride, procaterol hydrochloride, tulobuterol hydrochloride, clenputerol hydrochloride, ketotifen fumarate and the like; antineoplastic drugs such as cyclophosphamide, fluorouracil, degafur, mitomycin C, procarbazine hydrochloride, doxifluridine, ranimustine and the like; topical anesthetics such as ethyl aminobenzoate, tetracaine hydrochloride, brocaine hydrochloride, dibucaine hydrochloride, oxybuprocaine hydrochloride, propitocaine hydrochloride and the like; hormone preparations such as propylthiouracil, thiamazole, metelonone acetate, estradiol, estriol, progesterone and the like; antihistamine agents such as diphenhydramine hydrochloride, chlorpheniramine maleate, promethazine, dyproheptadine hydrochloride, diphenylpyraline hydrochloride and the like; anticoagulants such as warfarin potassium, ticlopidine hydrochloride and the like; anticonvulsive agents such as atropine methylbromide, scopolamine and the like; general anesthetics such as thiopental sodium, pentobarbital sodium and the like; hypnotics or analgesics such as bromovalenylurea, amobarbital, phenobarbital and the like; antiepileptic agents such as phenytoin sodium and the like; analeptics or stimulant drugs such as methamphetamine hydrochloride and the like; antidizziness drugs such as difendol hydrochloride, betahistine mesylate and the like; psychoneurotic agents such as chlorpromazine hydrochloride, thioridazine, meprobamate, imipramine hydrochloride, chlordiazepoxide, diazepam, risperidone, paliperidone, olanzapine, aripiprazole, paroxetine, duloxetine and the like; muscle relaxants such as suxamethonium hydrochloride, eperisone hydrochloride and the like; autonomic agents such as neostigmine bromide, bethanechol chloride and the like; antiparkinson agents such as amantadine hydrochloride, rotigotine, ropinirole and the like; anti-Alzheimer-type dementia drugs such as donepezil, galanthamine, memantine, rivastigmine and the like; diuretics such as hydroflumethiazide, isosorbide, furosemide and the like; vasoconstrictors such as phenylephrine hydrochloride and the like; respiratory stimulants such as lobeline bromide, dimorpholamine, naloxone hydrochloride and the like; peptic ulcer therapeutic agents such as glycopyrronium bromide, proglumide, cetraxate hydrochloride, cimetidine, spizofurone and the like; cholagogues such as ursodesoxycholic acid, osalmid and the like; urogenital and anus agents such as hexamine, sparteine, dinoprost, ritodrine hydrochloride, oxybutynin, tolterodine, solifenacin, darifenacin and the like; agents for parasitic skin diseases such as salicylic acid, ciclopirox olamine, coroconazole hydrochloride and the like; skin softeners such as urea and the like; vitamins such as calcitriol, thiamine hydrochloride, riboflapin sodium phosphate, pyridoxine hydrochloride, nicotinamide, panthenol, ascorbic acid and the like; mineral preparations such as calcium chloride, potassium iodide, sodium iodide and the like; hemostatic drugs such as ethamsylate drug; agents for liver diseases such as tiopronin and the like; agents for habitual intoxication such as cyanamide and the like; therapeutic agents for gout such as colchicine, probenecid, sulfinpyrazone and the like; diabetic agents such as tolbutamide, chlorpropamide, glymidine sodium, glypuzole, puformin hydrochloride, insulin and the like; antibiotics such as benzylpenicillin potassium, propicillin potassium, cloxacillin sodium, ampicillin sodium, bacampillicin hydrochloride, carbenicillin sodium, cephaloridine, cefoxitin sodium, erythromycin, chloramphenicol, tetracycline, kanamycin sulfate, cycloserine and the like; chemotherapeutic agents such as isocyanide, pyrazinamide, ethionamide and the like; narcotics such as morphine hydrochloride, codeine phosphate, cocaine hydrochloride, pethidine hydrochloride, fentanyl citrate and the like; and the like. The salt is not limited to those mentioned above, and various salts or free forms can be used.

The content of a drug or a pharmaceutically acceptable salt thereof in the transdermal absorption preparation is not particularly limited. In consideration of dispersibility in the adhesive layer and drug releaseability, the content is preferably 1 wt% - 30 wt%, more preferably 2.5 wt% - 25 wt%, most preferably 4 wt% - 20 wt%, in the adhesive layer. In consideration of the peel resistance and the like of a patch during bathing, not more than 15 wt% is preferable.

The adhesive sheet for adhesion to the skin and the transdermal absorption preparation of the present invention are constituted by extending an adhesive layer having the above-mentioned constitution on a support.

The "support" in the present invention is not particularly limited and those used widely can be employed. For example, stretchable or non-stretchable woven fabric or non-woven fabric of polyethylene, polypropylene and the like, films of polyethylene, polypropylene, ethylene vinyl acetate copolymer, vinyl chloride and the like, foamed supports of urethane, polyurethane and the like can be mentioned. These may be used alone, or plural kinds thereof may be laminated and uses. To prevent accumulation of static electricity on the support, as well as to achieve good anchor property to the adhesive layer, a non-woven fabric or woven fabric containing an antistatic agent can be used.

Moreover, the transdermal absorption preparation of the present invention may contain excipient, antioxidant, flavor, colorant and the like as an optional component.

Examples of the excipient to be used in the present invention include silicon compounds such as silicic anhydride, light anhydrous silicic acid, hydrated silicate and the like, cellulose derivatives such as ethylcellulose, methylcellulose, hydroxypropylcellulose, hydroxypropylmethylcellulose and the like, water-soluble polymers such as polyvinyl alcohol and the like, aluminum compounds such as dried aluminum hydroxide gel, hydrated aluminum silicate and the like, kaolin, titanium oxide and the like.

Examples of the antioxidant to be used in the present invention include dibutylhydroxytoluene, ascorbic acid, tocopherol, tocopherol ester derivative, butylhydroxyanisole, 2-mercaptobenzimidazole and the like.

### [Examples]

The present invention is explained in more detail in the following by referring to Examples and Comparative Examples, which are not to be construed as limitative.

### [Examples 1 - 13, Comparative Examples 1 - 4] Preparation of adhesive sheet for adhesion to the skin

According to the formulation shown in Table 1, each component constituting an adhesive layer was weighed. First, styrene-isoprene-styrene block copolymer (SIS)/styrene-isoprene block copolymer (SI) mixture ("KRAYTON D1119" manufactured by KRATON POLYMERS INC.), "Quintac3520" manufactured by Nippon Zeon Co., Ltd., and "JSR SIS5505", "JSR SIS5229" manufactured by JSR Corporation were dissolved in 230 parts by weight of toluene per 100 parts by weight of the mixture. To the aforementioned solution were added liquid paraffin ("BENOL", "KAYDOL", "Hydrobrite 550PO", "Hydrobrite HV" manufactured by Sonneborn Limited) and the mixture was mixed and stirred to give a coating liquid forming an adhesive layer. In the Table, "D1111" is an abbreviation of "KRATON D1111", "D1119" is an abbreviation of "KRATON D1119", "QTC3520" is an abbreviation of "Quintac3520", "5505" is an abbreviation of "JSR SIS5505", and "5229" is an abbreviation of "JSR SIS5229".

The above-mentioned coating fluid was applied to a silicone-treated poly(ethylene terephthalate) (PET) film (release liner), and adjusted such that the adhesive layer after drying was 300 µm. After drying for 1 hr in an oven at 80°C, a PET film (support) was laminated on a surface of the adhesive layer, and cut into 15 cm × 30 cm to give the object adhesive sheet. The SIS/SI ratios in the Table are weight ratios.

In Comparative Examples 3 and 4, each component constituting the adhesive layer was measured according to the formulation shown in Table 1, and sheets were prepared according to the above-mentioned method. In Comparative Example 3, sufficient adhesiveness was not obtained and in Comparative Example 4, the adhesive layer could not be maintained and evaluation was not possible.

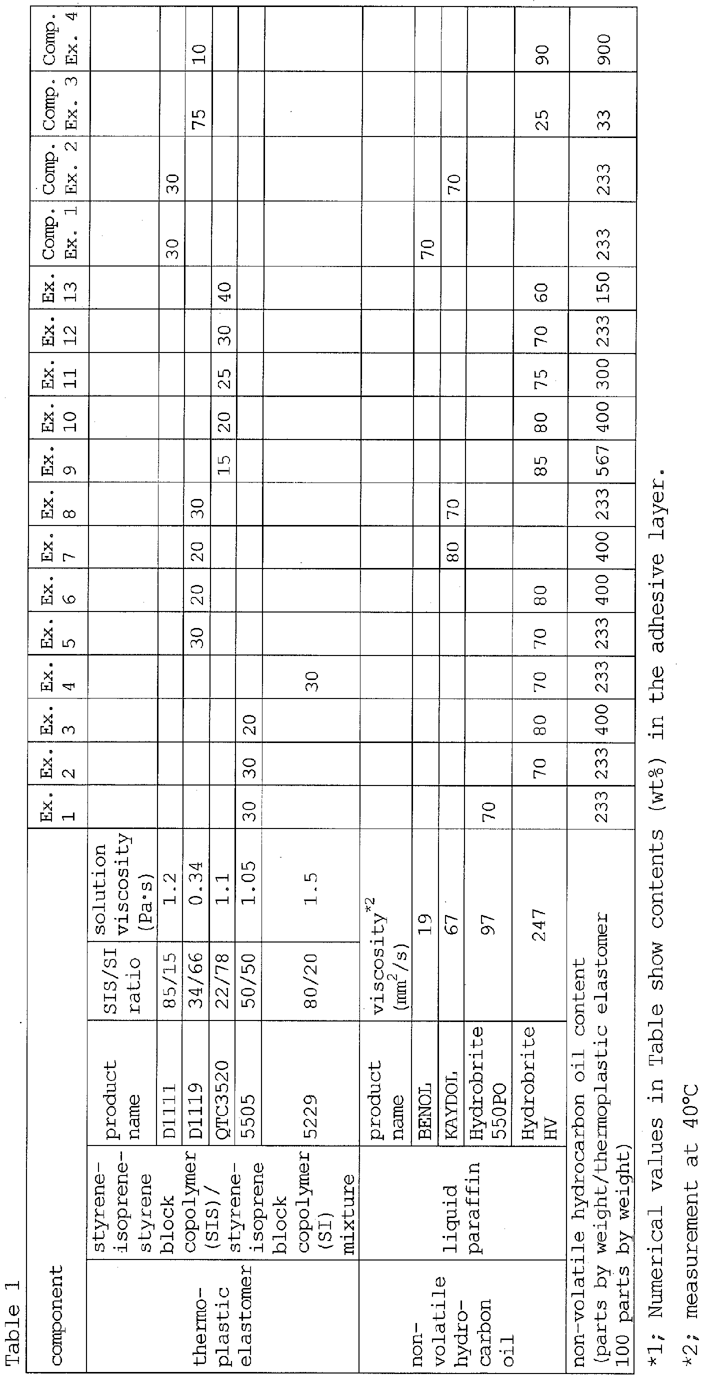

### [Comparative Example 5] Preparation of silicone adhesive sheet

According to the method of the Example of WO 2007/064407, a coating solution having a composition after drying of silicone adhesive ("Bio-PSA Q7-4301" manufactured by Dow Corning Corporation) (98.9 wt%), silicone oil (1.0 wt%), and vitamin E (0.1 wt%) was prepared and applied to a Teflon-treated poly(ethylene terephthalate) (PET) film (release liner) such that the weight per unit area after drying was 90 g/m² and dried.

A PET film (support) was laminated on a surface of the adhesive layer, and cut into 15 cm × 30 cm to give the object adhesive sheet.

### [Experimental Example 1] Adhesive property tests

### <peel strength>

An adhesive sheet cut in 25 mm x 100 mm was adhered to a stainless (SUS304) plate, and a stress on detachment in the 180° direction at a speed of 300 mm/min was measured.

### <ball tackiness>

A 1/32 inch - 1 inch ball was rolled, after a 100-mm approach, down on an inclined plane with an angle of inclination of 30°, onto which an adhesive sheet cut in 100 mm width was adhered, and the nominal diameter of the largest ball that stopped on the adhesive sheet for not less than for 5 seconds was measured.

### <holding force>

An adhesive sheet cut in 25 mm x 100 mm was adhered to a stainless (SUS304) plate, a 25 g load was hung in the 90° direction for 60 min, and the detached distance was measured.

### <protrusion>

The end portion of the adhesive sheets prepared in the Examples and Comparative Examples was compressed with a finger from the top of the support, and the level of protrusion was evaluated according to the following criteria.
A: no protrusion of adhesive layer even when compressed
B: almost no protrusion of adhesive layer even when compressed
C: on compression, adhesive layer is deformed and protrudes from support but is restored after release of compression
D: on compression, adhesive layer is deformed and protrudes from support and is not restored easily even after release of compression

### <degree of detachment in bathing>

The adhesive sheets prepared in the Examples and Comparative Examples were punched out in a circular shape with a diameter of 36 mm, adhered to the chest of five healthy volunteers, and the degree of detachment in bathing was evaluated according to the following criteria.
A: no detachment in 5 volunteers
B: end portion was detached in 1 - 2 volunteers but did not fall off
C: adhesive sheet fell off in 1 - 2 volunteers
D: fell off in 3 or more volunteers

### [Experimental Example 2] primary skin irritation test

Three days before the start of adhesion, dorsal hair of kbs:JW female domestic rabbit (17-week-old) was shaven with an electric clipper, and the adhesive sheet of Example 1 and a commercially available rivastigmine-containing patch which were each cut into a 2.5 cm square were adhered to the skin (n=3). Oil paper was placed thereon to cover the adhesion site, an underlap tape (manufactured by Nichiban Co., Ltd.) was wound from the chest to the whole abdomen, and a jacket for domestic rabbit (BJ03, manufactured by Bioresearch Center Co., Ltd.) was set thereon. After fixing for 24 hr, the sample was removed, and the level of skin irritation reaction was evaluated based on the method described in J. Pharmacol. Exp. Ther. 82, 377-390 (1944) for 1 hr, for 24 hr, for 48 hr and for 72 hr after the removal.

That is, at each of the above-mentioned times, erythema and eschar formation and edema formation were evaluated according to the following evaluation criteria, and scored. An average of respective evaluation points was determined, the primary evaluation value was calculated, and an average of the average evaluation value at each of the above-mentioned times was determined for each domestic rabbit and taken as a primary irritation index (P.I.I.). The P.I.I. value was 0 at the lowest and 8 at the highest, and the values are divided into 4 categories of primary skin irritation reaction shown in Table 2.

### <evaluation criteria of skin irritation reaction>

### [formation of erythema and escahr]

no erythema; 0 score
very slight (barely perceptible level of) erythema; 1 score
well-defined erythema; 2 scores
moderate to severe erythema; 3 scores
severe erythema to eschar formation of level preventing erythema scoring; 4 scores

### [formation of edema]

no edema; 0 point
very slight (barely perceptible level of) edema; 1 score
slight edema (edges of area well defined by definite raising); 2 scores
moderate edema (raised approximately 1 mm); 3 scores
severe edema (raised more than 1 mm and extending beyond exposure area); 4 scores

**Table 2**

| category of skin primary stimulation reaction | P.I.I |
|---|---|
| no stimulation | 0 - 0.4 |
| weak stimulation | 0.5 - 1.9 |
| moderate stimulation | 2 - 4.9 |
| strong stimulation | 5 - 8 |

Using the above-mentioned test method, Examples 1 - 13, Comparative Examples 1 - 2, Comparative Example 5, and commercially available adhesive preparations ("MOHLUS TAPE L40 mg" manufactured by Hisamitsu Pharmaceutical Co., Inc., "Loxonin tape 100 mg" manufactured by DAIICHI SANKYO COMPANY LIMITED) were evaluated, and the results are shown in Table 3.

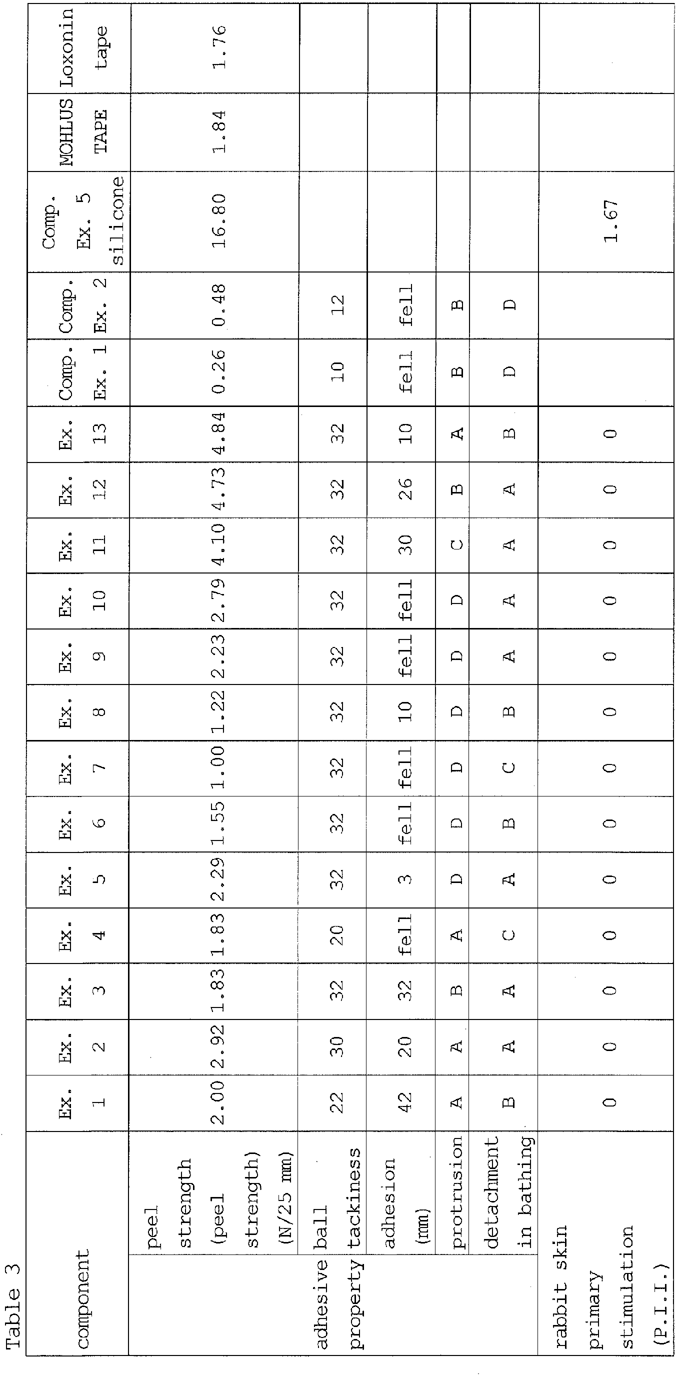

From Table 3, it was clarified that each adhesive sheet of the Examples of the present invention showed an adhesive property lower than that of a silicone adhesive sheet, but appropriate adhesive property which is of the equivalent level as or a higher level than that of commercially available adhesive preparations. On the other hand, the results of the skin irritation were low when compared to those of silicone adhesive sheets. Particularly, when liquid paraffin having high kinematic viscosity was used, high adhesiveness was shown.

### [Examples 14 - 27, Comparative Examples 6 - 9] Preparation of transdermal absorption preparation

According to the formulation shown in Table 4, each component constituting an adhesive layer was weighed. First, styrene-isoprene-styrene block copolymer (SIS)/styrene-isoprene block copolymer (SI) mixture ("KRAYTON D1119" manufactured by KRATON POLYMERS INC.), "Quintac3520" manufactured by Nippon Zeon Co., Ltd., and "JSR SIS5505", "JSR SIS5229" manufactured by JSR Corporation were dissolved in 230 parts by weight of toluene per 100 parts by weight of the mixture. To the aforementioned solution were added liquid paraffin ("BENOL", "KAYDOL", "Hydrobrite 550PO", "Hydrobrite HV" manufactured by Sonneborn Limited), various additives and a medicament, and the mixture was mixed and stirred to give a coating liquid forming an adhesive layer. In the Table, "D1111" is an abbreviation of "KRATON D1111", "D1119" is an abbreviation of "KRATON D1119", "QTC3520" is an abbreviation of "Quintac3520", "5505" is an abbreviation of "JSR SIS5505", and "5229" is an abbreviation of "JSR SIS5229".

The above-mentioned coating fluid was applied to a silicone-treated poly(ethylene terephthalate) (PET) film (release liner), and adjusted such that the rivastigmine content of the adhesive layer after drying was 1.8 mg/cm². After drying for 1 hr in an oven at 80°C, a PET film (support) was laminated on a surface of the adhesive layer, and cut into 15 cm × 30 cm to give the object transdermal absorption preparation. The SIS/SI ratios in the Table are weight ratios.

In Comparative Examples 6 and 7, each component constituting the adhesive layer was measured according to the formulation shown in Table 4, and transdermal absorption preparations were prepared according to the above-mentioned method. In Comparative Example 6, sufficient adhesiveness was not obtained and in Comparative Example 7, the adhesive layer could not be maintained and evaluation was not possible.

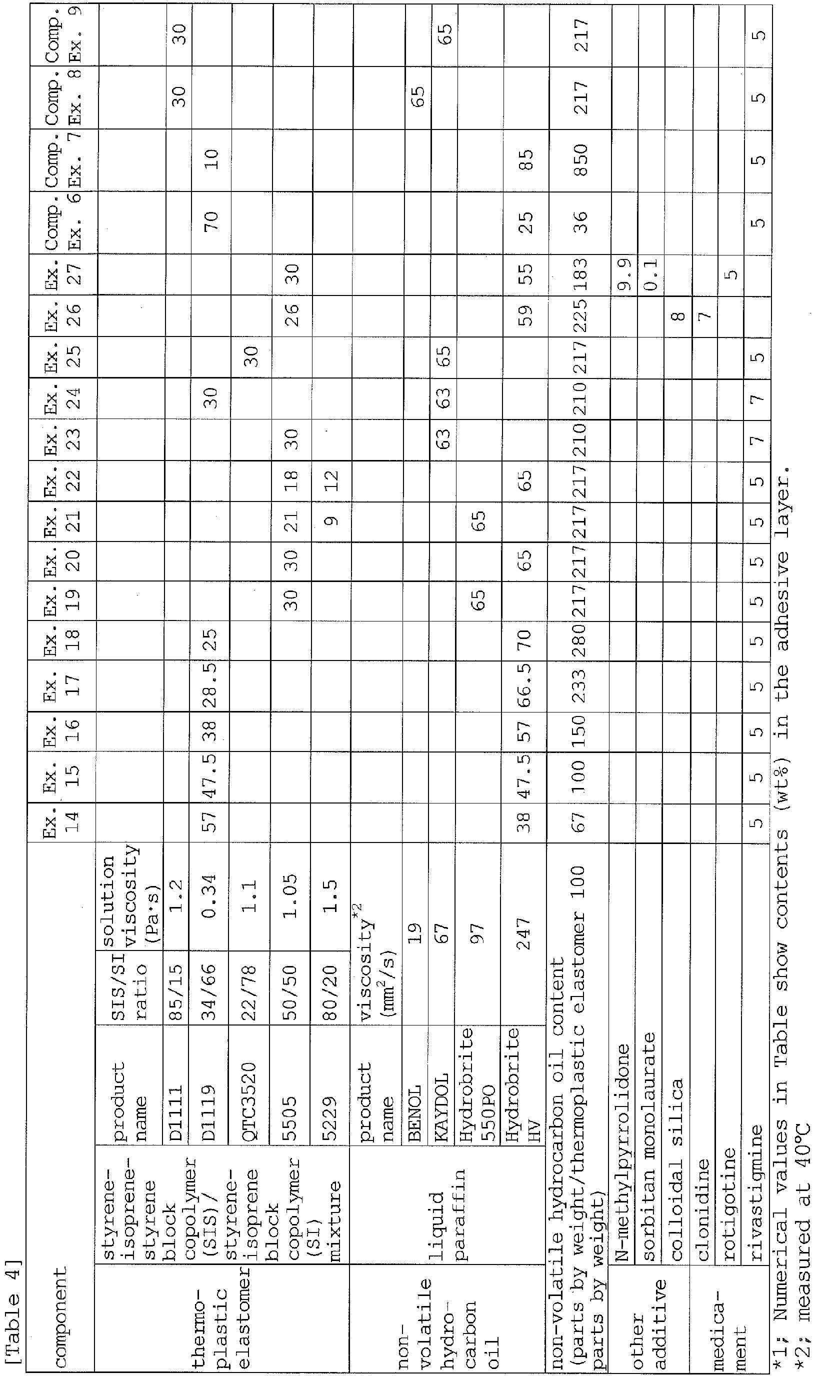

### [Experimental Example 3] In vitro skin permeability test

According to the method described in WO 2006/093139, the skin extracted from the abdomen of male Wister rat (5-week-old) was set on a vertical Franz diffusion cell. Each transdermal absorption preparation of Examples 9 - 21 and commercially available transdermal absorption preparation was punched out into a circular shape (area 1.0 cm²) to give a sample, which was adhered to the skin of the rat in the diffusion cell (n=3). As a receptor side, 10% by volume ethanol saline was used, and the rivastigmine content of the receptor solution was quantified over time by high performance liquid chromatography (HPLC).

The amount of the drug that permeated the rat skin was determined for 24 hr after adhesion of the sample.

The results of evaluation using the above-mentioned test method are shown in Tables 5 - 7.

**[Table 5]**

| component | | Ex. 14 | Ex. 15 | Ex. 16 | Ex. 17 | Ex. 18 | Ex. 19 | Ex. 20 | Ex. 21 | Ex. 22 | Ex. 23 | Ex. 24 | Ex. 25 | Comp. Ex. 8 | Comp. Ex. 9 | commercially available adhesive preparation containing rivastigmine |
|---|---|---|---|---|---|---|---|---|---|---|---|---|---|---|---|---|
| adhesive property | peel strength (peel strength) (N/25 mm) | 4.38 | 4.65 | 3.63 | 2.29 | 3.38 | 2.74 | 3.41 | 2.82 | 2.91 | 5.43 | 1.2 | 1.07 | 0.31 | 0.52 | |
| | ball tackiness | 32 or more | 32 or more | 30 | 32 or more | 32 or more | 30 | 32 or more | 26 | 28 | 32 or more | 22 | 24 | 12 | 14 | |
| | adhesion (mm) | 4 | 0 | 8 | 5 | 2 | 50 | 35 | 50 | 45 | 30 | fell | - | fell | fell | |
| | protrusion | A | A | B | C | D | B | A | A | A | B | A | B | B | B | |
| | detachment in bathing | D | D | B | B | A | B | A | C | B | A | C | C | D | D | |
| skin permeation amount of medicament in 24 hr after adhesion of sample (µg/cm2) | | 370 | 385 | 400 | 410 | 410 | 365 | 360 | 355 | 350 | 360 | 505 | 521 | | | 360 |
| rabbit skin primary stimulation (P.I.I.) | | | 0 | | | 0 | 0 | 0 | 0 | 0 | 0 | 0 | 0 | | | 2.92 |

**[Table 6]**

| | | Ex. 24 | commercially available adhesive preparation containing clonidine |
|---|---|---|---|
| adhesive property | peel strength (peel strength) (N/25 mm) | 3.55 | |
| | ball tackiness | 28 | |
| | adhesion (mm) | 30 | |
| | protrusion | A | |
| | detachment in bathing | A | |
| skin permeation amount of medicament in 24 hr after adhesion of sample (µg/cm2) | | 36 | 27 |

**[Table 7]**

| | | Ex. 25 | commercially available adhesive preparation containing rotigotine |
|---|---|---|---|
| adhesive property | peel strength (peel strength) (N/25 mm) | 3.61 | |
| | ball tackiness | 30 | |
| | adhesion (mm) | 25 | |
| | protrusion | A | |
| | detachment in bathing | A | |
| skin permeation amount of medicament in 24 hr after adhesion of sample (µg/cm2) | | 68 | 41 |

From Tables 5 - 7, it was clarified that each transdermal absorption preparation of the Examples of the present invention showed small skin irritation and superior adhesive property, while showing drug releaseability equivalent to or higher than that of commercially available transdermal absorption preparations.

### Industrial Applicability

The adhesive sheet for adhesion to the skin of the present invention shows low skin irritation and appropriate adhesiveness, and a transdermal absorption preparation utilizing same maintains them and is superior in the drug releaseability, and is possibly utilized for improving the properties of existing transdermal absorption preparations, as well as for the development of a new transdermal absorption preparation.

This application is based on a patent application No. 2013-273767 filed in Japan and an international application PCT/JP2013/066273.

## Claims

1. An adhesive sheet for adhesion to the skin, comprising an adhesive layer formed on a support, wherein
the adhesive layer comprises
at least a thermoplastic elastomer, and
a non-volatile hydrocarbon oil in an amount exceeding 50 parts by weight and not more than 800 parts by weight per 100 parts by weight of the elastomer, and
the thermoplastic elastomer is a mixture of a triblock copolymer and a diblock copolymer, wherein the content of the diblock copolymer in the mixture is not less than 20 wt%, and
wherein the non-volatile hydrocarbon oil has a kinematic viscosity of not less than 80 mm²/s at 40°C,
wherein the content of the thermoplastic elastomer in the adhesive layer is not less than 8 wt% and not more than 66 wt%,
wherein the thermoplastic elastomer is a mixture of a styrene-isoprene-styrene block copolymer and a styrene-isoprene block copolymer, and
wherein the adhesive layer optionally further comprises a tackifier, and the content of the tackifier in the adhesive layer is not more than 2 wt%, and
wherein, when the adhesive layer comprises the tackifier, the tackifier is selected from the group consisting of rosin-based resin, polyterpene resin, cumarone-indene resin, petroleum-based resin, terpene-phenol resin, and alicyclic saturated hydrocarbon resin.

2. The adhesive sheet according to claim 1, wherein a 25 wt% toluene solution of the thermoplastic elastomer has a solution viscosity of not less than 0.5 Pa·s at 25°C.

3. The adhesive sheet according to claim 1 or 2, wherein the content of the diblock copolymer in the mixture is not less than 30 wt%.

4. The adhesive sheet according to claim 2 or 3, wherein a 25 wt% toluene solution of the thermoplastic elastomer has a solution viscosity of not less than 0.7 Pa·s at 25°C.

5. The adhesive sheet according to any one of claims 1 to 4, wherein the content of the non-volatile hydrocarbon oil in the adhesive layer is not less than 23.5 wt% and not more than 88 wt%.

6. The adhesive sheet according to any one of claims 1 to 5, wherein the adhesive layer is free of a tackifier.

7. A transdermal absorption preparation comprising the adhesive sheet according to any one of claims 1 to 6, and a drug or a pharmaceutically acceptable salt thereof, which is contained in the adhesive layer of the sheet.

## Patentansprüche

1. Klebefolie zum Haften auf der Haut, die eine auf einem Träger gebildete Klebstoffschicht umfasst, wobei
die Klebstoffschicht umfasst:
wenigstens ein thermoplastisches Elastomer; und
ein nichtflüchtiges Kohlenwasserstofföl in einer Menge von über 50 Gewichtsteilen und nicht mehr als 800 Gewichtsteilen pro 100 Gewichtsteile des Elastomers; und
das thermoplastische Elastomer ein Gemisch aus einem Triblockcopolymer und einem Diblockcopolymer ist, wobei der Gehalt des Diblockcopolymers in dem Gemisch nicht geringer als 20 Gew.-% ist; und
wobei das nichtflüchtige Kohlenwasserstofföl eine kinematische Viskosität von nicht weniger als 80 mm²/s bei 40 °C aufweist;
wobei der Gehalt des thermoplastischen Elastomers in der Klebstoffschicht nicht weniger als 8 Gew.-% und nicht mehr als 66 Gew.-% beträgt;
wobei das thermoplastische Elastomer ein Gemisch aus einem Styrol-Isopren-Styrol-Blockcopolymer und einem Styrol-Isopren-Blockcopolymer ist; und
wobei die Klebstoffschicht gegebenenfalls weiterhin einen Klebrigmacher umfasst und der Gehalt des Klebrigmachers in der Klebstoffschicht nicht mehr als 2 Gew.-% beträgt; und
wobei, wenn die Klebstoffschicht den Klebrigmacher umfasst, der Klebrigmacher aus der Gruppe ausgewählt ist, die aus Kolophoniumharz, Polyterpenharz, Cumaron-Inden-Harz, Harz auf Erdölbasis, Terpen-PhenolHarz und alicyclischem gesättigten Kohlenwasserstoffharz besteht.

2. Klebefolie gemäß Anspruch 1, wobei eine 25-Gew.-%ige Toluollösung des thermoplastischen Elastomers eine Lösungsviskosität von nicht weniger als 0,5 Pa·s bei 25 °C aufweist.

3. Klebefolie gemäß Anspruch 1 oder 2, wobei der Gehalt des Diblockcopolymers in dem Gemisch nicht geringer als 30 Gew.-% ist.

4. Klebefolie gemäß Anspruch 2 oder 3, wobei eine 25-Gew.-%ige Toluollösung des thermoplastischen Elastomers eine Lösungsviskosität von nicht weniger als 0,7 Pa·s bei 25 °C aufweist.

5. Klebefolie gemäß einem der Ansprüche 1 bis 4, wobei der Gehalt des nichtflüchtigen Kohlenwasserstofföls in der Klebstoffschicht nicht weniger als 23,5 Gew.-% und nicht mehr als 88 Gew.-% beträgt.

6. Klebefolie gemäß einem der Ansprüche 1 bis 5, wobei die Klebstoffschicht frei von einem Klebrigmacher ist.

7. Präparat für transdermale Resorption, umfassend die Klebefolie gemäß einem der Ansprüche 1 bis 6 und einen Wirkstoff oder ein pharmazeutisch annehmbares Salz davon, der bzw. das in der Klebstoffschicht der Folie enthalten ist.

## Revendications

1. Feuille adhésive pour l'adhésion sur la peau, comprenant une couche adhésive formée sur un support, dans laquelle
la couche adhésive comprend
au moins un élastomère thermoplastique, et
une huile hydrocarbonée non volatile en une quantité qui est supérieure à 50 parties en poids et qui n'est pas supérieure à 800 parties en poids pour 100 parties en poids de l'élastomère, et
l'élastomère thermoplastique est un mélange d'un copolymère tribloc et d'un copolymère dibloc, où la teneur en copolymère dibloc dans le mélange n'est pas inférieure à 20 % en poids, et
dans laquelle l'huile hydrocarbonée non volatile a une viscosité cinématique qui n'est pas inférieure à 80 mm²/s à 40°C,
dans laquelle la teneur en élastomère thermoplastique dans la couche adhésive n'est pas inférieure à 8 % en poids et n'est pas supérieure à 66 % en poids,
dans laquelle l'élastomère thermoplastique est un mélange d'un copolymère à bloc de styrène-isoprène-styrène et d'un copolymère à bloc de styrène-isoprène, et
dans laquelle la couche adhésive comprend en outre éventuellement un agent adhésif, et la teneur en l'agent adhésif dans la couche adhésive n'est pas supérieure à 2 % en poids, et
dans laquelle, lorsque la couche adhésive comprend l'agent adhésif, l'agent adhésif est choisi dans le groupe consistant en une résine à base de colophane, une résine de polyterpène, une résine de cumarone-indène, une résine à base de pétrole, une résine de terpène-phénol, et une résine hydrocarbonée saturée alicyclique.

2. Feuille adhésive selon la revendication 1, dans laquelle une solution de toluène à 25 % en poids de l'élastomère thermoplastique a une viscosité en solution qui n'est pas inférieure à 0,5 Pa·s à 25°C.

3. Feuille adhésive selon la revendication 1 ou 2, dans laquelle la teneur en le copolymère dibloc dans le mélange n'est pas inférieure à 30 % en poids.

4. Feuille adhésive selon la revendication 2 ou 3, dans laquelle une solution de terpène à 25 % en poids de l'élastomère thermoplastique a une viscosité en solution qui n'est pas inférieure à 0,7 Pa·s à 25°C.

5. Feuille adhésive selon l'une quelconque des revendications 1 à 4, dans laquelle la teneur en huile hydrocarbonée non volatile dans la couche adhésive n'est pas inférieure à 23,5 % en poids et n'est pas supérieure à 88 % en poids.

6. Feuille adhésive selon l'une quelconque des revendications 1 à 5, dans laquelle la couche adhésive est exempte d'un agent adhésif.

7. Préparation d'absorption transdermique comprenant la feuille adhésive selon l'une quelconque des revendications 1 à 6, et un médicament ou un sel pharmaceutiquement acceptable de celui-ci, qui est contenu dans la couche adhésive de la feuille.
